# EUROPEAN PATENT SPECIFICATION

(11) **EP 2 173 261 B1**
(45) Date of publication and mention of the grant of the patent: **08.08.2018**
(21) Application number: 08763585.0
(22) Date of filing: 18.06.2008
(51) Int. Cl.: A61B 17/20

(54) **APPARATUS FOR ULTRASOUND TISSUE TREATMENT**
GERÄT ZUR ULTRASCHALLBEHANDLUNG VON GEWEBE
APPAREIL POUR LE TRAITEMENT DES TISSUS PAR ULTRASONS

(30) Priority: 26.07.2007 US 951966 P
(43) Date of publication of application: 14.04.2010
(73) Proprietor: Syneron Medical Ltd., 20692 Yoqneam Illit (IL)
(72) Inventor: ROSENBERG, Avner, 36578 Bet Shearim (IL)
(74) Representative: FRKelly
(86) International application number: PCT/IL2008/000827
(87) International publication number: WO 2009/013729

(56) References cited:
- US-A- 4 131 021
- US-A- 6 113 558
- US-B1- 6 391 023
- US-B1- 6 500 141
- US-B1- 6 623 430
- US-B2- 6 662 054
- US-B2- 7 052 493

## Description

### TECHNOLOGY FIELD

The present device is in the field of ultrasound tissue treatment and, in particular, adipose tissue treatment for cosmetic and aesthetic purposes.

### BACKGROUND

Reduction of subcutaneous fat layers, or adipose tissue, is an aesthetic treatment for which there is a growing demand. Among the different physical therapies available, the application of ultrasound is emerging as a leading adipose tissue removal and body shaping technology. Methods associated with this technology are based on the delivery of a dose of electromagnetic or ultrasound energy through the skin of a recipient into the subcutaneous adipose tissue to a volume of tissue to be treated. It is believed that US Patent Nos. 6,500,141, 5,143,063, 5,507,790, 6,071,239, 6,113,558, United States Patent Publication No. 2004/0106867, and US Patent Application 11/335,181 to the assignee of the present application reflect the current state of this technology.

In order to deliver to the recipient the ultrasound dose, a caregiver attaches an applicator, including ultrasound transducers, to the recipient's skin and transmits the desired dose of energy. The caregiver, however, has no indication of the quality of coupling and transmission of the ultrasound through the tissue, and has no information on the interaction of the ultrasound energy with the tissue. There is no information on how successful was the treatment in the change of the aggregation state (composition) of the adipose tissue, or when the introduction of ultrasound into the target volume may discontinue and treatment of the next volume may begin. Known technologies for treatment control are based on the measurement of ultrasound reflections. From these reflections they derive the information on the interaction of the ultrasound energy with the tissue. However, the reflected energy does not include sufficient information on the processes taking place in the target tissue volume.

In order to increase the ultrasound power concentrated in a target volume a number of transducers are focused on the same volume and operated simultaneously and continuously. The interaction between the power of ultrasound waves emitted by these transducers, emitting one in the direction of the others, is a phenomenon that sets emitted power limit. Each transducer has an upper limit of the power density, and the power density at the transducer is the sum of the power emitted by the transducer and the power impinged upon it from the other transducers.

For faster development of the ultrasound application technology there is a need to resolve these and other problems and enable a better control over the ultrasound treatment process.

### BRIEF LIST OF DRAWINGS

The method and apparatus disclosed are herein presented, by way of nonlimiting examples only, with reference to the accompanying drawings, wherein like numerals depict the same elements throughout the text of the specifications.
Figure 1 is a schematic illustration of an apparatus for tissue treatment in accordance with one exemplary embodiment.
Figures 2A and 2B are schematic illustrations of some exemplary embodiments of the applicator for tissue treatment.
Figure 3 is a schematic illustration of a typical transmitted and induced signal monitored at a transducer operating according to an exemplary embodiment of the method.
Figure 4 is a schematic illustration of an apparatus for tissue treatment in accordance with an additional exemplary embodiment of the method.
Figure 5 is a schematic illustration of an additional exemplary embodiment of the applicator for tissue treatment.
Figure 6 is a schematic illustration of an applicator for tissue treatment in accordance with a further exemplary embodiment of the method.
Figure 7 is a cross section of the applicator for tissue treatment of Figure 6.

### DETAILED DESCRIPTION OF THE EXEMPLARY EMBODIMENTS

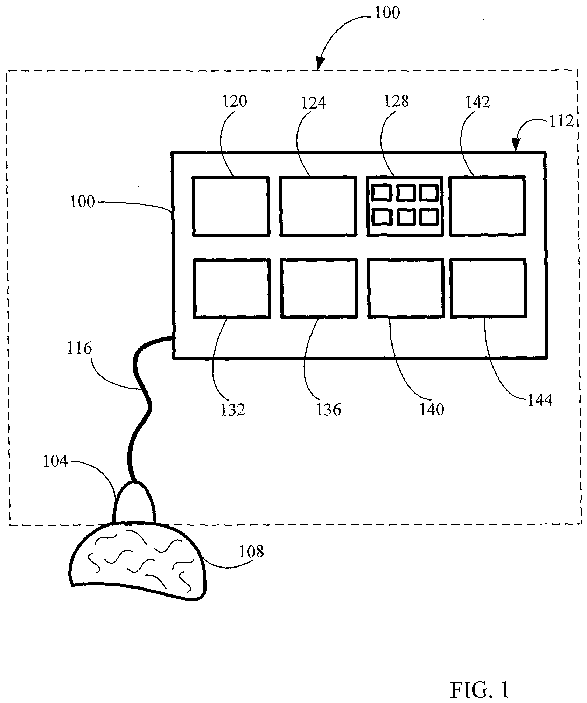

The invention is as defined in the appended claims. Reference is now made to Figure 1, which is a schematic illustration of an apparatus for tissue treatment in accordance with an exemplary embodiment. Apparatus 100 applies to a recipient tissue 108 with the help of applicator 104 ultrasound energy. As used herein, the term "tissue treatment" includes skin, dermis, adipose tissue treatment, and such procedures as fat tissue destruction, inducing fat necrosis, inducing fat apoptosis, fat redistribution, adipocyte (fat cell) size reduction, and cellulite treatment. Control unit 112 via a harness 116 governs operation of applicator 104. The control unit 112 inter alia includes an ultrasound driver 120 for electrically driving the ultrasound transducers located in applicator 104, a processor 124 for monitoring and controlling various functions of apparatus 100. Control unit 112 may have an input device, such as a keypad 128 that allows the caregiver or apparatus operator to input to the processor 124 different commands and desired treatment parameters. Such parameters may be frequency of the ultrasound, pulse duration, pulse repetition rate, intensity of the ultrasound energy to be directed to the treated tissue, RF intensity, vacuum time, and vacuum intensity.

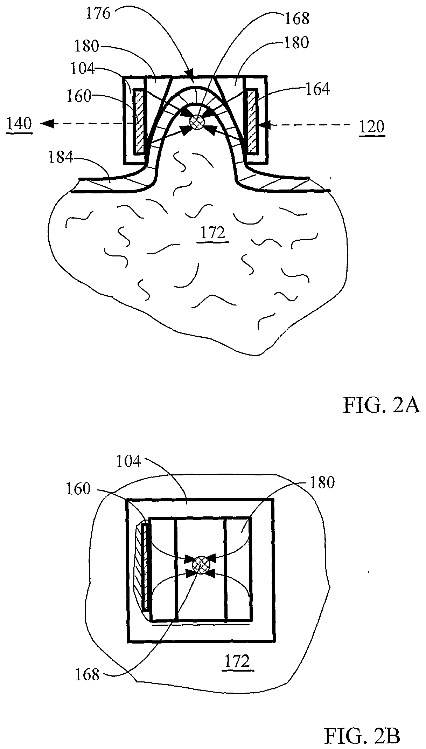

Control unit 112 may optionally include cooling means 132 for cooling applicator 104 and the section of skin surface during treatment. Cooling means 132 may be a refrigeration unit that cools a cooling fluid. The cooled fluid flows from cooling means 132 to applicator 104 via tubing, which may be part of harness 116. A vacuum pump 136 generates pressure below atmospheric pressure and causes a region of the tissue to protrude above the surround surface into the inner section of applicator 104. A vacuum hose embedded in harness 116 connects pump 136 to applicator 104. A display 142 for visual representation of the treatment process parameters may be included in control unit 120. An optional Radio Frequency (RF) generator 144 may be located in control unit 112. Generator 144 provides RF energy to applicator 104 if needed. Included in control unit 112 is a signal processing and analyzing unit 140, which may be implemented in hardware and/or software for receiving a feedback from applicator 104, processing and analyzing the feedback and communicating updated instructions to applicator 104. Figure 2 is a schematic illustration of an exemplary embodiment of the applicator for tissue treatment. Applicator 104 contains at least one ultrasound transducer 160 capable of emitting ultrasound power and at least one ultrasound transducer 164, which can receive the emitted and transmitted through protruding tissue ultrasound power. It is well known in the art that almost all types of ultrasound transducers can be used for both transmitting and receiving ultrasound energy. Accordingly, transducers 160 and 164 can be similar or identical and both of them may operate in either emitting or receiving mode. Ultrasound driver 120 provides electric power to ultrasound transducers 160 and 164. As used herein, the term "transducer" includes ultrasound emitter and ultrasound receiver.

Applicator 104 is adapted for application to the tissue of recipient 108 in a region of tissue to be treated. Applicator 104 may, in addition to transducers 160 and 164, optionally include one or more pairs of RF electrodes directing RF current supplied by generator 144 into the treated tissue. (Some of the applicator elements are omitted for clarity of the explanation.) When RF electrodes are included in applicator 104, processor 124 may monitor the electrical impedance between electrodes and determine the RF coupling quality. Processor 124 may also monitor the effectiveness of the treatment by monitoring variations of temperature in the tissue, since the electrical conductivity of the tissue is temperature dependent.

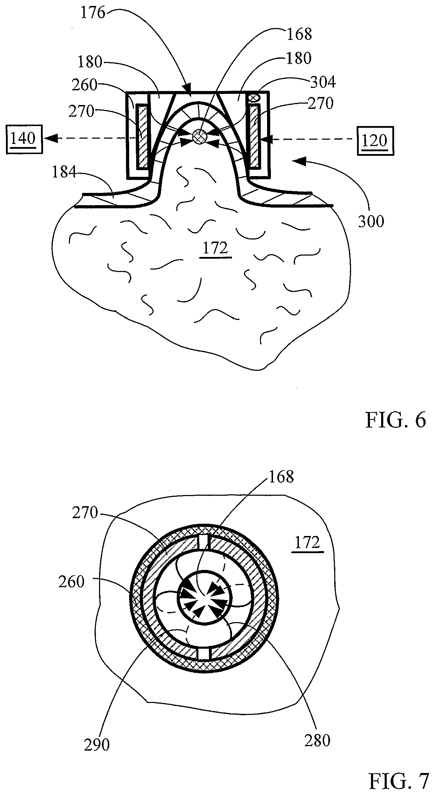

Figures 2A and 2B illustrate ultrasound transducers 160 and 164 as a pair of planar transducers, although it should be clear that they may be of curved and phase array types. As illustrated in Figure 6, the curved transducers may have a shape or be arranged such as to encircle the treated tissue. The number of transducers is not limited to two, and additional ultrasound transducers (or receivers) being in contact with the tissue protrusion 176 and emitting ultrasound waves such that they propagate from one transducer to another through the protruded tissue may be added to the applicator.

The 11/335,181 application inter alia exploits the high flexibility of tissue, and generates a protrusion 176 out of the body surface. This increases the area of the skin/tissue for delivering energy into the target tissue volume and improves the efficiency of the treatment. Negative pressure (vacuum) provided by pump 136 (Figure 1) to applicator 104 creates in the inner section of applicator 104 tissue 172 protrusion 176. Alternatively, mechanical manipulations of tissue 172 may create the protrusion. A pair of ultrasound transducers 160 and 164 is attached to that protrusion and the transducers radiate typically, in a direction parallel to the undisturbed skin surface, or at least as close as possible to that optimal angle. This allows concentrating of larger amounts of energy in the target volume 168, while much less radiation arrives at deeper body tissue. Optionally, wedge shaped ultrasound coupling means 180 having acoustical properties similar to those of soft tissues may be used to improve ultrasound to soft tissue coupling efficiency. Numeral 184 indicates upper tissue layers such as skin or dermis.

Applicator 104 is configured to deliver ultrasound energy to a region of subcutaneous tissue to be treated such as to generate a sufficient energy concentration for creating the desired treatment effects. A single transducer can, however, deliver a limited amount of ultrasound energy termed herein as the rated amount of ultrasound energy. There is also a limit to the degree of focusing and the size of the focusing volume of the ultrasound energy. US Patent Application 11/335,181 to the assignee of the present application teaches use of at least a pair of transducers, such as transducers 160 or 164, focused to the same target volume 168 of the treated tissue 172.

Transducers 160 and 164 can be operated simultaneously to deliver energy at the same target tissue volume 168 to increase the amount of energy coupled to the volume. In one embodiment both transducers are emitting ultrasound at the same frequency and with fixed relative phases. In such case, ultrasound velocity fields combine. Ultrasound power density is proportional to the square of absolute value of the sound velocity. Therefore, for two transducers radiating equal power densities, one can expect the power density at the target tissue to be up to four times the power emitted by a single transducer. Practically, this theoretical limit cannot be reached since each transducer has a limited power rating. When the ultrasound wave emitted by the first transducer and transmitted through the tissue in protrusion 176 reaches the second transducer, it interferes with the ultrasound wave emitted by the second transducer. The second transducer cannot handle the power density which it emits and the additional power density arriving from the first transducer. As noticed above, each transducer has a limited power rating. This rating reduces the ultrasound power, which can be emitted by the second transducer and vice versa.

According to an exemplary embodiment of the apparatus and method, operation of the transducers in a pulsed mode alleviates this problem. The duration of the pulse should be such that when the ultrasound wave front emitted by the first transducer arrives at the second transducer, the second transducer has already finished emitting and transmitting ultrasound. Pulses and, in particular, short pulses are never ideal square pulses. They always have a rise time and fall time and a further optimization of the pulse length could be done to obtain maximum power as well as maximum energy per pulse in the tissue. A textbook definition of pulse length or pulse duration is the width between half power points, or what is called the -3dB points. Accordingly, for the optimal situation it is possible to set the half power (-3dB) width of the pulse to be equal to, or smaller than, the transition time of the ultrasound from one transducer to another.

The author of the present method has experimentally proved that short pulses are at least as good for tissue treatment as longer ones and produce the same ultrasound energy-tissue interaction results and, in particular, fat tissue cell destruction. In order to compare the influence of different pulse durations, the same average ultrasound power ([peak power]x[duty cycle]) was maintained in all of the tests.

An important advantage of using short ultrasound pulse is that the power density at the outer layers 184 is small compared with power density at the fat tissue. According to one embodiment, two or more ultrasound transducers 160 emitting radiation of the same frequency from opposite sides of the protrusion into the tissue 172, are focused at the same volume 168. If the pulse (half power width) is shorter than the transition time between the transducers, the ultrasound waves can combine at the focal volume to four times the power of a single transmitting transducer. Thus, high power density exists only at the focal volume of the protrusion where the fat tissue resides. The ultrasound power density at the outer layers 184 is substantially lower than the power density in the focal volume, due to the differences in arrival times of the ultrasound pulse. The peak of the power of the pulse arrives at that layer from the nearest transducer before the peak of power arriving from the other transducer. This avoids any potential damage to the outer layers 184.

If the duration of the transmitted pulse is shorter than the time required to the ultrasound emitted by the first transducer to travel through the tissue to the second transducer, then almost all of the pulses emitted and transmitted through the tissue wave arrive at the second transducer after it had finished emitting ultrasound and may serve as a receiver. If a pulse equals twice the transition time, half of the pulse is received after the end of the transmission. The signal induced in the second transducer may, for example, be voltage induced in one transducer by the ultrasound emitted by the other transducer. The signal might be sampled and analyzed by means well known in the art. Thus, the use of short pulses enables real time diagnostic of the ultrasound transmission through the tissue and analysis of the ultrasound interaction with the treated tissue, based on the same transducers used for the power transmission into the tissue, without the need for additional specific receiver transducers.

A numerical example provides better feeling of the pulse durations discussed. For example, the distance between the transducers may be 40 to 50 mm. The average sound velocity in the tissue is about 1500m/sec. The pulse transition time would be about 27 to 33 microseconds. Therefore, if pulse duration is shorter than this time and the pulse is transmitted simultaneously by both transducers when the beginning of the ultrasound wave transmitted by one transducer arrives at the second one, this has already stopped transmitting and can function as a receiver. In practice ultrasound pulses are not ideal rectangular, and have rise and fall time which have to be considered when selecting the optimal pulse length. While the best way is to use a pulse equal to or shorter than the transmission time, one can use a longer pulse and sample only the trailing part of the pulse, which arrives after the end of the transmission.

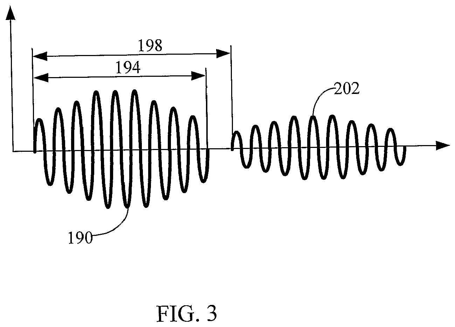

There is no need to use identical transducers. The receiving transducer should be capable of receiving at least part of the frequency spectrum transmitted by the transmitting transducer. Figure 3 shows an example of a typical image of signals at each of the transducers 160 or 164. Numeral 190 marks the transducer driving voltage. It continues for a certain time marked by numeral 194. Upon completion of the transmission time 198 the ultrasound wave from the other transducer arrives, and induces at the transducer signal 202, which is sampled and communicated to the signal processing and analyzing module 140. Typically, the amplitude of the induced signal 202 will be lower than that of the transmitted signal due to ultrasound propagation losses and limited transducer efficiency. Processing of both signals gives valuable data on the transmitted and the received ultrasound. The signal sampling on the transducer can be done by any standard techniques, such as capacitive coupling, magnetic coupling, or resistive coupling.

Ultrasound wave transmitted through the protruding tissue carries with it information on the interaction of the ultrasound with the tissue and, in particular, on the quality of coupling of the ultrasound from the transducers to the tissue and the transmission through the tissue. Analysis of the signals induced at the receiving transducer by the ultrasound pulses emitted by the transmitting transducer may include at least one of the following parameters: the signal transmission time, or what may be termed as the time taken for the signal emitted by the first transducer to arrive at the second transducer; the peak signal intensity at the front of the received pulse, and intensity fluctuations following the front of the pulse.

The time of arrival is a direct measure of the average sound velocity inside the tissue. The average sound velocity gives indications on the treated tissue aggregate state. For example, since the sound velocity on the skin is higher than in the fat, thicker skin will increase the average sound velocity and reduce the pulse arrival time. Sound velocity in tissues is known to be temperature dependent. Therefore, measurement of sound velocity can give data on the change of tissue temperature and the efficiency of the treatment associated with it. Monitoring tissue temperature is important for controlling the treatment and avoiding unwanted damage to tissue. Heating of tissue is generated by the ultrasound energy and/or by optional introduction of RF energy, which is applied to the treated tissue as disclosed in the US Patent Application No. 11/335,181 to the same assignee. Very large variation of ultrasound travel time from a nominal and known value will indicate an undesired path of ultrasound propagation.

The peak intensity at the front of the pulse is a direct indication that the transmitting transducer is working properly and that the coupling of ultrasound to tissue is correct and, accordingly, the transmission through tissue is good.

Experiments conducted by the author of the suggested method indicated that when the ultrasound is applied in continuous mode and the threshold of fat destruction is reached, the induced signal intensity begins to fluctuate. When ultrasound is applied to the tissue in a pulse mode, the fluctuations start after the front of the pulse. Therefore, measurement of intensity fluctuations following the received pulse front is an indication of the effectiveness of the treatment. In addition, it was observed that when the fat tissue destruction process was completed, the transmitted signal fluctuations stopped, and transmission through tissue became high and stable. This received pulse behavior provides good real time information at the start and the end of the fat tissue destruction process.

This information, together with additional information extracted from the processing of the induced signal is communicated to processor 124 (Figure 1) and may provide data to be employed for real time, active monitoring of the ultrasound radiation with tissue coupling process. Corrective actions may include change of operational parameters of the transmitted ultrasound or of the vacuum suction which creates the protrusion (this parameter has an effect on the coupling), or of the RF energy if included in the applicator (RF energy change the tissue temperature, which can affect the ultrasound propagation and interaction effects in the tissue.) The operational parameters of the transmitted ultrasound are at least: pulse peak power, pulse length, pulse repetition rate, relative phase between transducers and focusing scheme if phased arrays are used. The peak ultrasound intensity may be set to exceed the threshold power for the desired tissue treatment effects, but should not to become too high to reduce the risk of collateral damage.

Based on the monitored signal analyses communicated to processor 124 by processing unit 140, processor 124 (Figure 1) may undertake corrective action and change automatically one or more of the above listed operational parameters. Such apparatus will operate in a closed loop mode and intervention of the caregiver or apparatus operator will be not required. Alternatively, the information extracted from the processing of the induced signal may be displayed on display 142 (Figure 1) providing the caregiver or apparatus operator with the possibility of manual operation of the apparatus. Manual change of ultrasound operational parameters is performed by entering them via keypad 128.

The real time ultrasound radiation with tissue coupling (or interaction) monitoring process is applicable to long pulse or continuously radiating transducers. In such case, one transducer emits ultrasound required for treatment and the other transducer serves as a receiver only for at least part of the time. The control system 124 can be set for time division of tasks such that for most of the time both transducers are transmitting, with no induced signal analysis of the transmitted energy. At the time that one of the transducers is not driven to emit ultrasound it may function as a receiver. The processing and analyses of the induced signal are executed in a similar way. However, part time signal monitoring does not provide information on full power density at the target tissue volume, since one of the transducers is not transmitting at the monitoring time. Application of phased array transducers can alleviate this problem and offer additional advantages for the treatment of tissue.

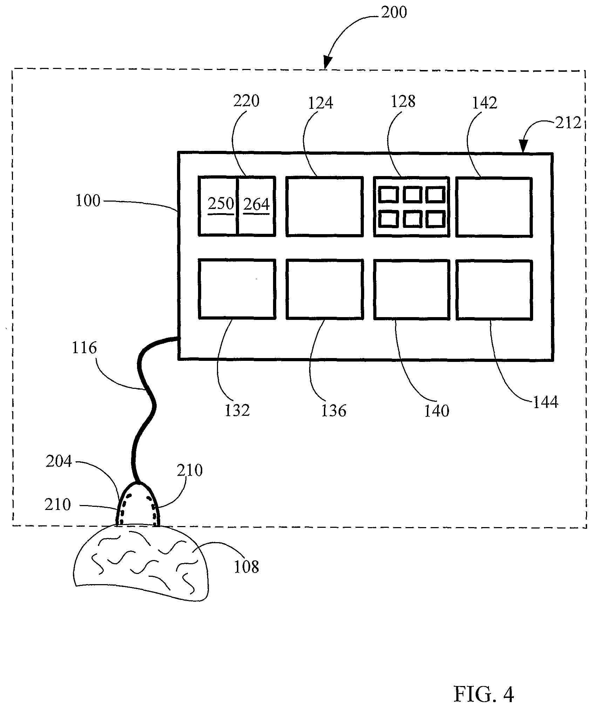

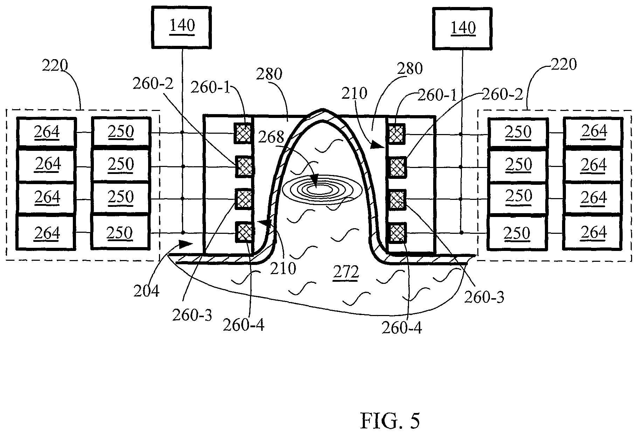

Figure 4 is a schematic illustration of another embodiment of the apparatus for tissue treatment in accordance with an exemplary embodiment of the method. Apparatus 200 has a number of features distinct from apparatus 100. Applicator 204 includes phased array ultrasound transducers 210. An independent driver channel 250 located in the drivers unit 220 of controller 212 drives each individual element 260-1 through 260-4 (Figure 5) of transducers 210. Phase generators 264 control the phase of each element 260. Control unit 212 controls the phasing program of elements 260. The phasing program is configured to obtain a focal volume 268 (Figure 5) at the desired location inside tissue 272.

Figure 5 is a schematic illustration of an additional exemplary embodiment of the applicator for tissue treatment. Applicator 204 includes phased array ultrasound transducers 210 positioned face to face. Transducers 210 deliver ultrasound waves into tissue 272. Optionally, coupling means 280 may be employed to improve ultrasound to tissue coupling. Tissue protrusion 226 is generated by vacuum suction or mechanical manipulation. Each transducer 210 is built from a plurality of elements 260. Although only four elements are shown in each transducer, their number and size may be chosen according to a known practice of phased array design. Independent driver channel 250 drives each element 260. Phase generators 264 control the phase of each element 260. Control unit 212 controls the phasing program of elements 260, such that they are focused in a target volume 268.

Two elements of each transducer, for example elements 2 and 3, are sampled. Any number of elements can be chosen for sampling and induced signal analysis. The more elements are selected, the more accurate is the transmission data. Sampling can be done by any known coupling means. The signal produced by the coupling means is fed to the processing and analyzing module 140 and processor 124. As explained earlier, processor 124 can automatically change treatment parameters, stop treatment in case of poor coupling, and/or display the information for a caregiver or operator decision. All the induced signal analyses described above are *mutatis mutandis* applicable to the phased array transducers.

Furthermore, the application of phased array transducers enables sophisticated analysis of the data received and better treatment control. For example, the operator may instruct the control system to perform a scanning of the focal volume. The control system computes the phases to get the focal volume at the required position. If the ultrasound transmission is sufficient for each focal volume, a specific set of voltages is expected at the sampled elements of the arrays. Any deviation from proper transmission will be noticed and can be analyzed to get data on the transmission. For example, elements 2 and 3 generate equal voltages when the focal point is aimed between them. Occasional shift of the focal volume could be detected by a difference in the voltage generated by elements 2 and 3. Short pulses are used with phased array transducers in a way similar to the uniform phase transducers. Phased arrays enable easier monitoring of the transmission with long pulses or continuous transmission. The control system 240 can select and sample a specific element for a desired time. During that time, the selected element will not be driven for transmission and used as a receiver. The loss of power at the focal tissue volume is small if the number of elements in the array is sufficiently large. For example, in a 32 elements array, one element not emitting will reduce the emitted radiation by 1/32 only. A more sophisticated control scheme can sample elements according to a time and position program.

The author of the present method has experimentally found that when the ultrasound pulses break the fat tissue cell an unarmed ear hears a sound. The signal is a type of "knocking" noise accompanying each ultrasound pulse. Figure 6 is a schematic illustration of an applicator for tissue treatment in accordance with a further exemplary embodiment of the method. Applicator 300 includes an acoustical microphone 304. The microphone collects audible sound from the treated tissue volume, and filters only those sounds, which are accompanying an ultrasound pulse. Transducers 270, as shown in Figure 7 could be curved, regular or phase array transducers that may have a shape or be arranged such as to encircle the treated tissue 172. Different operating sections of phase array transducers 270 shown in Figure 7, which is a cross section of the applicator for tissue treatment of Figure 6, may be operated such as to concentrate the ultrasonic field in a common target focal volume 168. Numerals 280 (solid lines) and numeral 290 (phantom lines) show ultrasonic field produced by transducers 270 and directed from different directions to be concentrated in focal volume 168.

The method and apparatus disclosed give the caregiver a clear indication of the quality of coupling and transmission of the ultrasound through the tissue, provide information on the interaction of the ultrasound energy with the tissue, and improve the quality of the treatment.

Induced signal power fluctuations provide information on how successful was the treatment in the change of the aggregation state of the adipose tissue, when the introduction of ultrasound into the target volume may discontinue and the treatment of the next volume may begin.

By using short pulses, high power density can be obtained in the tissue to be treated without exceeding the power rating of the transducers.

Another benefit of the short pulse is that the ultrasound power density at the outer tissue layers is substantially lower than the power density in the focal volume. This avoids any potential damage to the outer tissue layers.

Short pulses enable transmitting and receiving of the same by a transducer, and separating the transmitted from received signals by their propagation time delay.

The application of phased arrays enables time-sharing between transmission and receiving tasks with negligible loss of power.

Occasional shift of the focal volume or other disturbances of ultrasound propagation could be detected by difference in the voltage generated by receiving elements of a phased array transducer.

The embodiments aspects and examples which do not fall within the scope of the claims are provided for illustrative purpose only and do not form part of the present invention. The invention is defined in the claims as follows.

## Claims

1. An apparatus (100/200) for ultrasound aesthetic treatment of soft tissue, said apparatus comprising:
an applicator (104) adapted to generate a protrusion (176) of said soft tissue, said applicator (104) comprising at least one ultrasound energy transmitting transducer (160) and at least one ultrasound energy receiving transducer (160) and said apparatus (100/200) further comprising a controller (212) driving said applicator (104) and a signal processing and analyzing unit (140), said apparatus (100/200) **characterized in that** the transducers (160) are positioned face-to-face on opposite sides of said protrusion (176), wherein the transducer (160) radiates ultrasound energy in a direction parallel to undisturbed skin surface and the signal processing and analyzing unit (140) processes the signal received by said receiver (160) of ultrasound energy and derives data to actively monitor the ultrasound with tissue coupling.

2. The apparatus according to claim 1, wherein the applicator generates the tissue protrusion by vacuum or mechanical means.

3. The apparatus according to claim 1, wherein said signal processing and analyzing unit is at least one of a software or hardware and wherein parameters of said received ultrasound pulse signal include at least one of the signal transition time, peak received intensity at the front of the pulse, intensity fluctuation following the front of the pulse, and phase shift.

4. The apparatus according to claim 1, wherein said transducer is one of a uniform phase transducer, curved transducer, or phased array transducer and wherein at least a section of said phased array transducer operates as a receiver and wherein the receiving section of said transducer provides a feedback on said treatment process to said controller and where based on said feedback the controller sets at least one of the ultrasound signal operating parameters.

5. The apparatus according to claim 1, wherein said applicator further comprises at least a pair of RF electrodes

6. The apparatus according to claim 1, wherein each of the transducers is operative upon completion of transmission time to receive an ultrasound wave from another transducer.

7. The apparatus according to claim 1, wherein the transducer is operative to operate in pulse mode and wherein the pulse duration is shorter than pulse transition time and the pulse is transmitted simultaneously by both transducers so and wherein the beginning of an ultrasound wave transmitted by one transceiver arrives at the other transceiver which has already stopped transmitting and functions as a receiver.

8. The apparatus according to claim 1, wherein each of said transducers is operative to focus ultrasound energy to the same target volume of treated tissue.

9. The apparatus according to claim 1, wherein the processor is operative to take corrective action and change automatically on or more operational parameters and wherein said parameter setting is performed in either automatic or manual mode based on the monitored signal analysis communicate thereto.

10. The apparatus according to claim 3, wherein the fluctuations of said received signal intensity indicate destruction of said fat tissue and absence of received signal power fluctuation indicates completion of the fat tissue destruction process.

## Patentansprüche

1. Vorrichtung (100/200) für ästhetische Ultraschallbehandlung von Weichgewebe, wobei die Vorrichtung Folgendes umfasst:
einen Applikator (104), der angepasst ist, eine Protrusion (176) des Weichgewebes zu erzeugen, wobei der Applikator (104) mindestens einen Ultraschallenergieübertragungswandler (160) und mindestens einen Ultraschallenergieempfangswandler (160) umfasst und wobei die Vorrichtung (100/200) ferner eine den Applikator (104) antreibende Steuerung (212) und eine Signalverarbeitungs- und -analysiereinheit (140) umfasst, wobei die Vorrichtung (100/200) **dadurch gekennzeichnet ist, dass** die Wandler (160) auf entgegengesetzten Seiten der Protrusion (176) einander gegenüberliegend positioniert sind, wobei der Wandler (160) Ultraschallenergie in einer zu der unbehandelten Hautoberfläche parallelen Richtung ausstrahlt und die Signalverarbeitungs- und -analysiereinheit (140) das von dem Empfänger (160) der Ultraschallenergie empfangene Signal verarbeitet und Daten ableitet, um den Ultraschall mit Gewebekopplung aktiv zu überwachen.

2. Vorrichtung nach Anspruch 1, wobei der Applikator die Gewebeprotrusion mittels Vakuum oder mechanischen Mitteln erzeugt.

3. Vorrichtung nach Anspruch 1, wobei die Signalverarbeitungs- und -analysiereinheit mindestens eines von einer Software oder Hardware ist und wobei Parameter des empfangenen Ultraschallimpulssignals mindestens eines von der Signalübergangszeit, maximaler empfangener Intensität an der Front des Impulses, Intensitätsschwankung nach der Front des Impulses und Phasenverschiebung beinhalten.

4. Vorrichtung nach Anspruch 1, wobei der Wandler eines von einem Einheitsphasenwandler, bogenförmigen Wandler oder Phased-Array-Wandler ist und wobei mindestens ein Abschnitt des Phased-Array-Wandlers als ein Empfänger betrieben wird und wobei der Empfangsabschnitt des Wandlers der Steuerung eine Rückmeldung über den Behandlungsvorgang bereitstellt und wobei auf der Basis der Rückmeldung die Steuerung mindestens einen der Ultraschallsignalbetriebsparameter einstellt.

5. Vorrichtung nach Anspruch 1, wobei der Applikator ferner mindestens ein Paar RF-Elektroden umfasst.

6. Vorrichtung nach Anspruch 1, wobei jeder der Wandler nach Beendigung der Übertragungszeit betriebsfähig ist, um eine Ultraschallwelle von einem anderen Wandler zu empfangen.

7. Vorrichtung nach Anspruch 1, wobei der Wandler betriebsfähig ist, um in einem Impulsmodus betrieben zu werden, und wobei die Impulsdauer kürzer als die Impulsübergangszeit ist und der Impuls von beiden Wandlern simultan übertragen wird und wobei der Beginn einer von einem Transceiver übertragenen Ultraschallwelle an dem anderen Transceiver, der das Übertragen bereits gestoppt hat und als ein Empfänger arbeitet, ankommt.

8. Vorrichtung nach Anspruch 1, wobei jeder der Wandler betriebsfähig ist, um Ultraschallenergie auf dasselbe Zielvolumen von behandeltem Gewebe zu fokussieren.

9. Vorrichtung nach Anspruch 1, wobei der Prozessor betriebsfähig ist, um Korrekturmaßnahmen vorzunehmen und einen oder mehrere Betriebsparameter automatisch zu ändern, und wobei die Parametereinstellung entweder im automatischen oder manuellen Modus auf der Basis der an ihn kommunizierten Analyse überwachter Signale durchgeführt wird.

10. Vorrichtung nach Anspruch 3, wobei die Schwankungen der Intensität empfangener Signale eine Zerstörung des Fettgewebes angeben und ein Fehlen einer Schwankung der empfangenen Signalleistung eine Beendigung des Fettgewebezerstörungsvorgangs angibt.

## Revendications

1. Appareil (100/200) de traitement esthétique par ultrasons de tissus mous, ledit appareil comprenant :
un applicateur (104) adapté pour générer une protubérance (176) desdits tissus mous, ledit applicateur (104) comprenant au moins un transducteur émettant une énergie ultrasonore (160) et au moins un transducteur récepteur d'énergie ultrasonore (160) et ledit appareil (100/200) comprenant en outre une unité de commande (212) qui commande ledit applicateur (104) et une unité de traitement et d'analyse de signaux (140), ledit appareil (100/200) étant **caractérisé en ce que** les transducteurs (160) sont placés face-à-face sur des côtés opposés de ladite protubérance (176), dans lequel le transducteur (160) rayonne une énergie ultrasonore dans une direction parallèle à une surface de peau non manipulée et l'unité de traitement et d'analyse de signaux (140) traite le signal reçu par ledit récepteur (160) d'énergie ultrasonore et dérive des données pour contrôler activement le couplage des ultrasons avec les tissus.

2. Appareil selon la revendication 1, dans lequel l'applicateur génère la protubérance de tissus par des moyens de vide ou mécaniques.

3. Appareil selon la revendication 1, dans lequel ladite unité de traitement et d'analyse de signaux est au moins l'un d'un logiciel ou d'un matériel et dans lequel des paramètres dudit signal d'impulsion ultrasonore reçu comportent au moins l'un d'un temps de transition de signal, d'une intensité maximale reçue au bord frontal de l'impulsion, d'une fluctuation d'intensité après le bord frontal de l'impulsion, et d'un déphasage.

4. Appareil selon la revendication 1, dans lequel ledit transducteur est l'un d'un transducteur de phase uniforme, d'un transducteur courbe, ou d'une sonde à déphasage et dans lequel au moins une section de ladite sonde à déphasage fonctionne en tant que récepteur et dans lequel la section de réception dudit transducteur fournit une rétroaction relative audit processus de traitement à ladite unité de commande et dans lequel en fonction de ladite rétroaction l'unité de commande règle au moins l'un des paramètres de fonctionnement du signal ultrasonore.

5. Appareil selon la revendication 1, dans lequel ledit applicateur comprend au moins une paire d'électrodes RF.

6. Appareil selon la revendication 1, dans lequel chacun des transducteurs fonctionne au terme d'un temps de transmission pour recevoir une onde ultrasonore depuis un autre transducteur.

7. Appareil selon la revendication 1, dans lequel le transducteur fonctionne pour travailler en mode impulsionnel et dans lequel la durée d'impulsion est plus courte que le temps de transition d'impulsion et l'impulsion est transmise simultanément par les deux transducteurs, et dans lequel le début d'une onde ultrasonore transmise par un émetteur-récepteur arrive au niveau de l'autre émetteur-récepteur qui a déjà arrêté de transmettre et fonctionne en tant que récepteur.

8. Appareil selon la revendication 1, dans lequel chacun desdits transducteurs fonctionne pour focaliser l'énergie ultrasonore vers le même volume cible de tissus traités.

9. Appareil selon la revendication 1, dans lequel le processeur fonctionne pour prendre une mesure correctrice et changer automatiquement un ou plusieurs paramètres opérationnels et dans lequel ledit réglage de paramètres est exécuté dans un mode automatique ou dans un mode manuel en fonction de l'analyse du signal contrôlé communiquée à celui-ci.

10. Appareil selon la revendication 3, dans lequel les fluctuations d'intensité dudit signal reçu indiquent une destruction desdits tissus gras et l'absence d'une fluctuation de puissance du signal reçu indique la fin du processus de destruction des tissus gras.
